# EUROPEAN PATENT SPECIFICATION

(11) **EP 2 033 604 B1**
(45) Date of publication and mention of the grant of the patent: **25.04.2012**
(21) Application number: 06757777.5
(22) Date of filing: 05.06.2006
(51) Int. Cl.: A61F 5/00

(54) **INTRAGASTRIC BALLOON ASSEMBLY**
INTRAGASTRISCHE BALLONANORDNUNG
ENSEMBLE BALLONNET INTRAGASTRIQUE

(43) Date of publication of application: 11.03.2009
(73) Proprietor: Garza Alvarez, José Rafael, México, D.F. 03100 (MX)
(72) Inventor: Garza Alvarez, José Rafael, México, D.F. 03100 (MX)
(74) Representative: ABG Patentes, S.L.
(86) International application number: PCT/MX2006/000044
(87) International publication number: WO 2007/142503

(56) References cited:
- WO-A-2005/023118
- WO-A-2005/112822
- WO-A1-2005/007231
- WO-A1-2005/023118
- US-A1- 2002 055 757
- US-A1- 2005 215 950
- US-A1- 2005 267 405

## Description

### FIELD OF THE INVENTION

This invention is directed to the gastroenterology and general surgery fields, especially to an intragastric balloon assembly for the treatment of people with severe or morbid obesity problems.

### BACKGROUND OF THE INVENTION

During the last two decades several intra and extra gastric devices have been developed to help reducing weight in people with severe or morbid obesity problems. Currently, most of the used devices include bands and balloons, which have had several modifications in order to improve their efficiency and avoid complications; however, problems such as gastric juice leakages and infection, gastric wall necrosis due to the device contact, etc, and in general situations representing problems for the patient and in many times failures or non-efficient weight loosing results, have not been solved. Likewise, the surgical techniques used for treating severe obesity are complicated and lead to morbi-mortality increase aside of the high cost for those treatments.

WO 2005/023118 A1, which represents the closest prior art, refers to an intragastric assembly comprising a balloon, an elongated element formed as a traction guide unitary with the balloon to join the balloon to the patient stomach wall, including a first end attached to the balloon and a second end joined to a handle; an inflating catheter joined to the balloon disposed to inflate the balloon; an inflating valve connected to the inflating catheter.

US 2005/0215950 A1 corresponds to the area of intravascular catheters. It refers to a balloon catheter which includes an elongated shaft, and an expandable member affixed to the distal portion of the elongated shaft such that a section of the elongated shaft extends through at least a portion of the expandable member.

US 2005/0267405 discloses a gastro-occlusive device, comprising a balloon disposable in a stomach cavity of a patient, and inflatable therein to occlude a portion of the volume of the stomach cavity, a gas flow tube coupled to a distal end thereof with the balloon and extending outwardly through a stomach wall for coupling with a gas source for selectively inflating the balloon. The balloon and the gas tube may be integrally formed as a single-piece item. Insertion of the device by laparoscopic technique is mentioned.

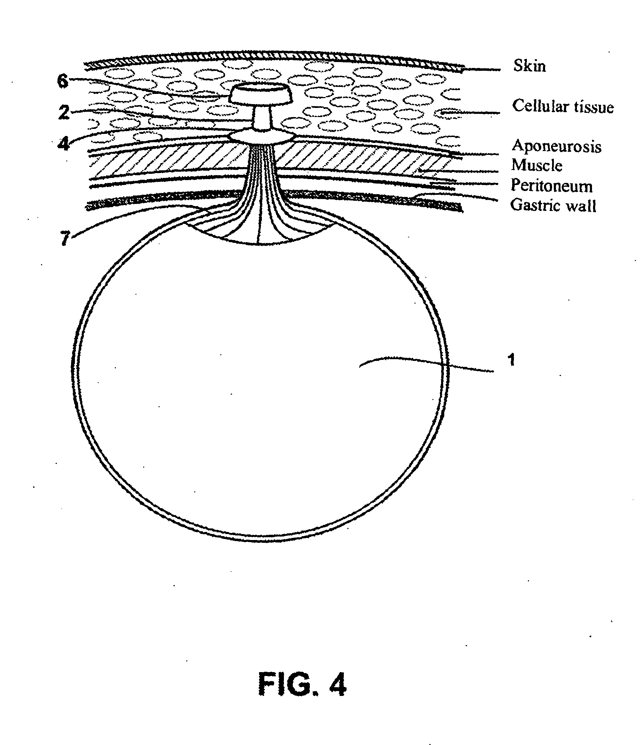

US 2002/0055757 discloses a space occupying device for deployment within a patient's stomach and methods of deploying and removing the device. The device can be deployed and/or removed thorough transesophageal approaches and/or thorough transesophageal and transabdominal approaches. In paragraph [0049], the description of Figure 4 mentions that the device includes a releasably attached inflator needle and an inflator tube.

WO 2005/112822 D5 discloses a balloon element sealed to a shaft element. Balloon element can be sealed to shaft element such that fluid communication with an interior of lumen of shaft element may cause the balloon element to expand or inflate. The shaft element may comprise a tubular element as known in the art. For example, shaft element may comprise a feeding tube. Any suitable catheter of feeding tube as known in the art may comprise shaft element. The gastric balloon is inserted within a stoma formed through a stomach of the patient.

An example of the related devices is shown in U. S. Patent application No. US 2003/0158569 A1, dated August 21, 2003, owned by Hussein Wazne, wherein an intragastric device for treating morbid obesity is disclosed, which is inserted by endoscopic path through the patient mouth, such device comprising a balloon or envelope having a specific nominal volume sealingly connected to connecting elements consisting of a disk forming support base for the balloon against the stomach wall; a flexible tube or catheter for connecting the balloon to a filling device, and fastening means being integrated to the tube or catheter, enabling said connecting elements to place or remove the balloon and to fix it either inside the patient stomach or subcutaneously, filling the balloon up to a predetermined nominal volume. Said document does not specify the material from which said device is made of; it is only described as made of plastics, such as polypropylene or of silicone, without further details. On the other side, it is disclosed that the balloon can be inflated within a range from 100 cc up to 1000 cc, which is a too wide range for the balloon elasticity, pointing out that it is inflated with air or a liquid, indistinctly. Likewise, it is disclosed that the device has two supporting plates, one of them inside of the stomach, in order to prevent possible leakages by pressing said plate into the stomach internal wall; the other outer supporting plate towards the aponeurosis or either towards the skin, without specifying how to place or fix the same. One of the disadvantages of said document is that an incision must be made in the patient during the balloon fixing to extract the catheter; and a great care is to be taken in said zone to perfectly seal said aperture, aside to use solutions or drugs to avoid a tissue infection or necrosis due to the contact thereof with the fixing means. It is not explained how this device is placed; it is just mentioned an endoscopy technique to introduce said balloon, but the way to carry it out is not disclosed. Also it is not disclosed how to introduce the catheter through the abdominal wall up to the stomach. Another important aspect to mention about the above cited US Patent application is that it discloses elements and characteristics very similar to the device protected by Mexican Patent No. 234,516 owned by the inventor of the present invention; wherein only the fixing axis is removed leaving the catheter along with two plates, being one of them internal, which instead of solving the problem of intragastric liquids leakages towards the abdominal wall, it may increase them or cause others damages; since the pressure of said plate over the mucosa and the gastric wall may cause injuries which can be from a tissues irritation and ulceration, up to the necrosis.

On the other hand, Patent document WO 02/40081 A2, by Thomas Bales et al, discloses an intragastric balloon device adapted to vary its volume several times without external assistance, which consist of an externally programmable inflation/deflation system, which allows that said balloon can have the desired size and can be placed in the stomach cavity. Said document does not describe fixing elements to prevent the balloon migration to the digestive tract, which may originate major complications, besides that no elements to avoid inconveniences after the balloon placing are disposed.

US Patent No. 6,755,869, of Kart Geitz describes an intragastric prosthesis for morbid obesity treatment, which consists of a porous weave of bioabsorbable filaments having an open mesh configuration is formed into an oblate shape having dimensions greater than the esophageal opening and gastric outlet of the stomach; said prosthesis is deployed in the stomach and is of a size to be retained in the proximate portion thereof for exerting pressure on the upper fundus. This document does not disclose exactly if there are prosthesis fastening elements, and according to the disclosure, said prosthesis can move from side to side in the stomach, causing a major problem, due to obstruction of any of the food inlet or outlet zones.

Regarding Mexican Patent No. 234,516, owned by the same inventor of the present invention, it combines three elements: an intragastric balloon, a valve to control the inflation and fixing elements of the assembly, to avoid the migration of the balloon inside the stomach. Although the proposed assembly provides an arrangement efficient for fixing said balloon in a secure way, the number of elements required to fix the assembly makes it relatively complicated either for its manufacture, placement and manipulation or for its high cost.

### SUMMARY OF THE INVENTION

This invention aims to provide an intragastric balloon assembly of simple design and construction to simplify its manufacture and reduce the related cost thereof.

Thus, it is an object of this invention to provide an intragastric balloon assembly capable of remain fixed in a patient stomach, in a preestablished place, during the treatment period thereof, without causing major complications, by fixing elements, which allow inflate the balloon with a fluid such as a gas or environment air.

Additionally, an object of this invention is to provide a means to firmly fasten the catheter to a supporting plate and to keep in this way the intragastric balloon assembly fixed.

Another object of this invention is to provide the balloon assembly with elements capable of preventing possible intragastric liquid leakages towards soft tissues of the abdominal wall.

Another further object of this invention is to provide a means of allowing a simple manner to control the balloon volume without major discomfort for the patient.

These and other objects of this invention are achieved by the intragastric balloon assembly as defined in claim 1, wherein the need of a rigid support, as disclosed in the Mexican Patent No. 234,516, owned by the same inventor, is avoided, of which function is carried out by the inflation catheter which is disposed at one of the balloon poles, and formed as a whole thereof.

The fixing system consist of a plain supporting plate with a single orifice through which an inflation catheter is conducted; which is subcutaneously fixed to the patient abdominal region and, in a preferred embodiment, it is also provided an element to firmly fasten the inflation catheter to said supporting plate , and consisting of a semi-rigid silicone washer or ring, including a small flange; said washer or ring is fixed with an appropriate adhesive causing in that way a suitable catheter tension and causing thereby a perfect attachment of the balloon, but without any pressure over the stomach wall; whereby the tissue in contact with the balloon assembly material is prevented from necrosis in said zone and, at the same time, a firm fastening of the catheter and assembly is obtained, thus allowing that the same remains fixed in the desired position.

In order to avoid intragastric liquid leakages towards the soft tissues of the abdominal wall, the invention provides, in the portion of the catheter coming from the balloon, and for about 3 cm of its length, a film of a material capable of promoting the fibrosis and the healing of the tissue, such as goretex or other similar substances.

On the other side, in order to inflate the intragastric balloon and control the volume thereof, after being placed in the patient, a multi-puncture valve is provided, connected to the balloon inflation catheter, which can be arranged on the supporting plate, thus having in this way an alternative element to fasten the catheter and maintaining the same under tension and thus, maintaining the balloon assembly fixed therein. All of these elements remain arranged under the skin and the subcutaneous cellular tissue.

In an additional embodiment, the multi-puncture valve can be disposed farther from the plate, by making another small incision in the skin and forming a tunnel in the cellular tissue, in order to conduct said catheter and connect it to said valve.

The present invention seeks to reduce the already known elements of the balloon assembly and to save material during its manufacturing; having as a consequence a reduced manufacturing cost, and an easier and faster placement can be obtained; and also the costs of a possible inflation modification will be certainty reduced, since the endoscopic procedure and the anesthetic sedation are suppressed.

### BRIEF DESCRIPTION OF FIGURES

The invention is illustrated in the enclosed drawings, as example, wherein:
FIGURE 1 shows the intragastric balloon assembly of this invention, in an exploded manner or as separated apart pieces.
FIGURE 2 shows a preferred embodiment of the intragastric balloon assembly, without the fixing elements.
FIGURE 3 shows the assembling manner of the intragastric balloon assembly along with the fixing elements; and
FIGURE 4 is a schematic view showing how the intragastric balloon remains placed in the patient body.

### DETAILED DESCRIPTION OF THE INVENTION

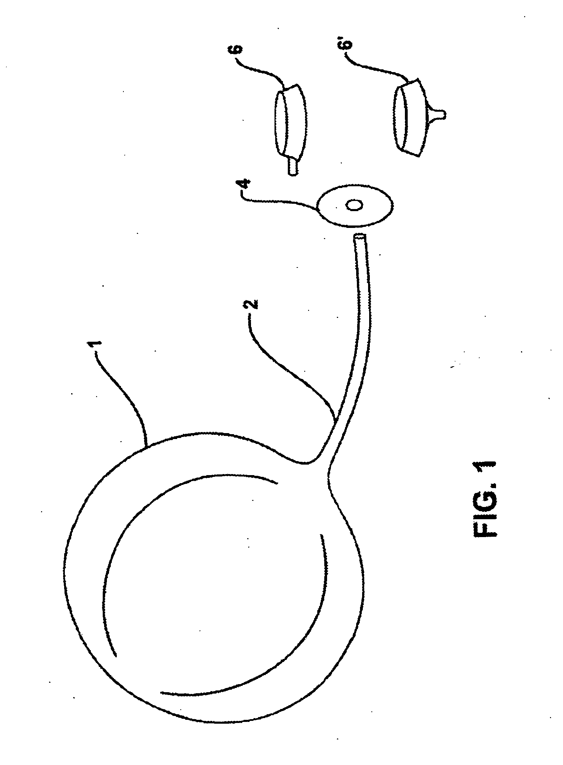

The present invention consist of an intragastric balloon assembly, which, as shown in Figure 1, includes a balloon 1 made of a material such as biomedical grade silicone with a volume capacity of 250 - 700 cm³; an inflation catheter 2, formed as a whole with the balloon, having an approximate length of 100 cm and a diameter between 1.5 to 2.0 mm, which is disposed at one of the balloon poles 1, which allows that a fluid or air can be introduced to inflate said balloon 1.

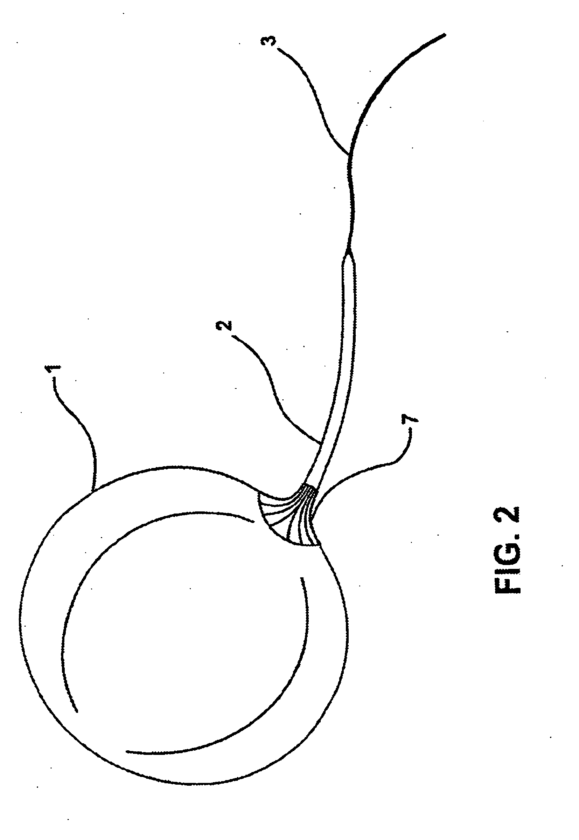

According to the invention, as illustrated in Figure 2, the inflation catheter 2 shows a traction guide 3 as an extension of Balloon 1 distal end; which will be useful to make traction from the balloon into the gastric cavity, and to lead catheter 2 through the stomach wall and other abdominal tissues. Said traction guide 3 can be made of a highly resistant silicone or polypropylene material.

The assembly of the invention also includes a supporting plate 4, which is usually a flat plate, made preferably of a medical grade material, such as silicone having preferably a thickness from 1.5 to 2.0 mm, with a preferred diameter from 4 to 5 cm, and showing an orifice through which the inflation catheter 2 is introduced. Said supporting plate 4 is placed and fixed on the anterior aponeurosis of the straight muscles of the abdomen, as shown in Figure 4, in the place previously determined by an endoscopy, and once the inflation catheter 2 is tense, it is fixed to the supporting plate 4, by means of a suitable adhesive, causing a catheter suitable tension, and a perfect attachment of the balloon, but without any pressure over the stomach wall; whereby the tissue in contact with the balloon assembly material is prevented from necrosis in said zone and at the same time, a firm fastening of the catheter and assembly is obtained, thus allowing that the same remains fixed in the desired position. In this way, the mentioned inflation catheter 2, along with the supporting plate 4, serve as fixing means of balloon 1 to the abdominal wall.

According to a preferred embodiment of the invention, a film 7 of a material capable of promoting tissue fibrosis and healing, such as goretex or other similar substances, at the balloon 1 zone in contact with the abdominal wall, as well as over a portion of catheter 2, preferably for about 3 cm of its length, in the portion of the catheter coming from balloon 1, as shown in Figures 2 to 4, in this way the adhesion to the balloon assembly of the gastric wall perforation edges is assured, preventing possible intragastric liquid leakages towards soft tissues through the abdominal wall perforation.

Likewise, the balloon assembly of this invention comprises a multi-puncture valve 6 to inflate and control the volume of balloon 1, connected to the distal end of the inflation catheter 2, in this way balloon 1 can be initially inflated through the valve once said balloon is placed inside the patient stomach, and balloon 1 inflation volume can be modified during the patient treatment, according to the results obtained during the treatment; that is, in relation to the patient loosing weight.

According to an embodiment of the invention, said multi-puncture valve 6 is connected to the inflation catheter 2 in a place close to where the supporting plate is disposed; so that the inflation catheter 2 can be maintained fixed, helping to fix and support balloon 1 in the patient stomach, as shown in Figure 4.

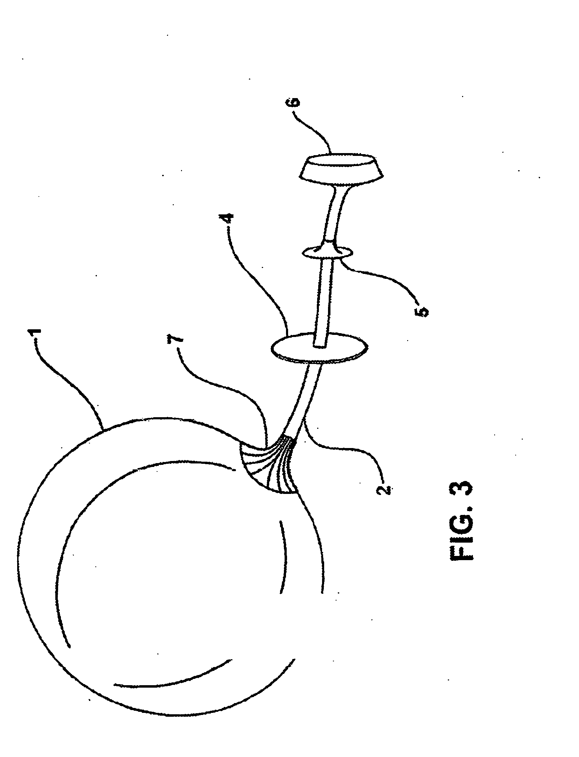

In an alternative embodiment, a washer or fixing ring 5 is provided as shown in Figure 3, having an orifice with a flange wherein the inflation catheter is introduced and attached to it with an appropriate adhesive causing the desired fixing of the elements to maintain the intragastric balloon 1 in a fixed position in the patient stomach.

Having fixed the inflation catheter 2, with the supporting plate 4 and fixing ring 5 assistance, the inflation valve 6 can be disposed in a remote place from the supporting plate 4, subcutaneously carrying the inflation catheter until connecting it with the valve 6.

In other embodiment of balloon 1 of the present invention, for inflation volume evaluation and control, it is additionally provided, a coating on balloon 1 or radio-opaque markers, which can be metal filaments included in the silicone (not shown), or other similar systems allowing observation thereof by means of radiological techniques. In this way, with a simple X-ray technique and local anesthesia, the volume of the balloon inside the patient can be observed and a modification of the same can be carried out by a needle puncture of valve 6, either for deflating or inflating said balloon 1, avoiding the need to use an endoscopy technique for evaluation and control.

The intragastric balloon of the present invention can be used in an endoscopic gastrostomy procedure comprising the steps of:
A) puncturing the abdominal wall in an endoscopy predetermined place, with a 2 mm trocar, reaching up to the stomach interior, which has been previously inflated by means of a puncture of the gastric wall up to the abdominal wall;
B) carrying out previously an incision from the skin point and cellular tissue to the aponeurosis;
C) introducing the distal end of the inflation catheter 2 of the intragastric balloon assembly, towards the stomach inner cavity, by means of an endoscope, holding said catheter 2 end with a clamp passing it through the 2mm trocar; once the catheter is passed, it is extracted up to the abdominal wall when the trocar is withdrawn, where the traction thereof can be effected as well as the balloon pulling which is located in the opposite end of the catheter, by the same endoscope, to be able to push and pull it until its final position in the stomach;
D) withdrawing the catheter to the abdominal wall;
E) Making traction of the catheter in the incision place, until said balloon (continuously monitored by endoscopy) be attached to the gastric wall, without excessive tension, avoiding the possible wall necrosis;
F) placing the supporting plate 4, by conducting the inflation catheter 2 through the orifice plate and supporting the entire intragastric balloon assembly;
G) joining the distal end of catheter 2 to the multi-puncture valve 6, in an immediate point to the place wherein the supporting plate 4 is disposed, so the inflation catheter 2 is maintained tense and fastened and, therefore, the fixing and support of said balloon 1 is obtained in the patient stomach.

In an alternative embodiment, the following steps are included:
G') placing a fixing washer 5, by passing the inflation catheter 2 through the orifice thereof, placing said fixing washer 5 on the supporting plate 4;
H) adhering the fixing elements (4 and 5) with a surgical adhesive, avoiding suture stitches or metal staples use;
I) joining the distal end of said catheter to the multi-puncture valve 6, and fixing said valve 6 subcutaneously.

In other embodiment, if the surgeon consider appropriate, it can be carried out the following:
I') Moving the inflation catheter 2 to a side to connect it to the multi-puncture valve 6, setting said valve 6 in a farther place from the fixing elements 4 and 5, making another small incision in the skin and tunneling the cellular tissue in order to pass said catheter 2 and connect it to said valve 6.

By means of this arrangement, the intragastric balloon assembly seeks to reduce the number of elements employed in the balloons actually in the market and those disclosed in the prior art. Moreover, this arrangement reduces the number of pieces to be manufactured for this purpose, making multi-puncture valves which are available in the market and easy to obtain, whereby the production costs will be lower. Likewise, the placing will be faster and simple, and also the cost of any possible modification of inflation will be certainly lower, since it is only effected by radiological control, without hospitalization, avoiding the endoscopic procedure which is indispensable in the balloon assembly of the prior patent of the same inventor.

It is important to notice that the efficient use results of the present invention, as the patients loosing weight it is equivalent to surgical or gastric bands procedures, avoiding the trauma of any of said procedures; and said efficiency is greater than the one of the current used balloons, only recommended for temporal treatments and with low tolerance and weight loosing effectiveness.

It will bye evident for those skilled in the art that several modifications and adaptations may be made to the invention herein described and illustrated, without separating from the scope thereof.

## Claims

1. An intragastric balloon assembly comprising an intragastric balloon (1), a traction element adapted to lead the balloon to the patient gastric cavity through the mouth and gastric tube thereof; an inflation catheter (2) formed as a whole with the balloon and, adapted to be passed through the stomach wall and abdominal tissues, and to inflate said intragastric balloon (1) with a fluid; fixing elements to fix said balloon (1) in a predetermined position within the patient gastric cavity, so avoiding the balloon migration; and an element to control the intragastric balloon inflation volume (6); wherein the ballon assembly has, as an only traction element, a traction guide (3) formed as an elongation of the inflation catheter free distal end and adapted to carry the balloon towards the gastric cavity and to lead said inflation catheter through the stomach wall and other abdominal tissues; wherein the fixing elements consist of a supporting plate (4) with a single orifice adapted to conduct the inflation catheter distal end, which is adapted to be fixed on the anterior aponeurosis of the straight muscles of the abdomen; and wherein the element for the inflation volume control, consists of an inflating multi-puncture valve (6), adapted to be connected to the inflation catheter (2) distal end, in a further point from the supporting plate (4), and is adapted to be fixed under the patient skin at the abdominal region.

2. Intragastric balloon assembly as claimed in claim 1, wherein the inflating multi-puncture valve (6) is adapted to be connected to the inflation catheter (2) in a place close to where the supporting plate is disposed, and to help as a fixing element to fix the inflation catheter to said supporting plate.

3. Intragastric balloon assembly as claimed in claim 1, further comprising a fixing washer (5) having an orifice with a flange, through which the inflation catheter (2) can pass, the fixing washer (5) adapted to be placed on the supporting plate, and the multi-puncture valve being disposed in a remote point from the supporting plate.

4. Intragastric balloon assembly as claimed in claims 1 to 3 wherein the balloon is made of biomedical grade silicone.

5. Intragastric balloon assembly as claimed in claims 1 to 4, further comprising a film of a material capable of promoting the fibrosis and the healing of the tissue, in the zone where the balloon and catheter are in contact with the abdominal wall.

6. Intragastric balloon assembly according to claims 1 to 5, further comprising a intragastric balloon radio-opaque coating for radiological evaluation of the balloon placed into a patient.

7. Intragastric balloon assembly according to claims 1 to 6, further comprising intragastric balloon radio-opaque markers for radiological evaluation of the balloon placed into a patient.

8. Intragastric balloon assembly as claimed in claim 7, wherein the radio-opaque markers are metal filaments included in the silicone.

## Patentansprüche

1. Magenballonanordnung, umfassend einen Magenballon (1), ein Zugelement zum Führen des Ballons durch den Mund und die Speiseröhre des Patienten in dessen Magen; einen Aufpumpkatheter (2), der einstückig mit dem Ballon ausgebildet ist und durch die Magenwand und Unterleibsgewebe geführt werden und den Magenballon (1) mit einer Flüssigkeit aufpumpen kann; Befestigungselemente zum Fixieren des Ballons (1) in einer vorbestimmten Lage innerhalb des Magens des Patienten, um **dadurch** ein Wandern des Ballons zu vermeiden; und ein Element (6) zur Steuerung des Aufpumpvolumens des Magenballons; wobei die Ballonanordnung als einziges Zugelement eine Zugführung (3) aufweist, die als Verlängerung des freien distalen Endes des Aufpumpkatheters ausgebildet ist und den Ballon zum Magen hin tragen und den Aufpumpkatheter durch die Magenwand und anderen Unterleibsgewebe führen kann; wobei die Befestigungselemente aus einer Trägerplatte (4) mit einer einzigen Öffnung bestehen, die das distale Ende des Aufpumpkatheters führen kann, welches an der vorderen Aponeurose der geraden Bauchmuskeln befestigt werden kann; und wobei das Element zur Steuerung des Aufpumpvolumens aus einem aufpumpenden Multipunktionsventil (6) besteht, das an einem Punkt hinter der Trägerplatte (4) mit dem distalen Ende des Aufpumpkatheters (2) verbunden und im Unterleibsbereich unter der Haut des Patienten fixiert werden kann.

2. Magenballonanordnung gemäß Anspruch 1, wobei das aufpumpende Multipunktionsventil (6) an einer Stelle in der Nähe der Trägerplatte mit dem Aufpumpkatheter (2) verbunden werden kann und als Befestigungselement die Fixierung des Aufpumpkatheters an der Trägerplatte unterstützen kann.

3. Magenballonanordnung gemäß Anspruch 1, die weiterhin eine Fixierscheibe (5) umfasst, die eine Öffnung mit einem Flansch aufweist, durch die der Aufpumpkatheter (2) geführt werden kann, wobei die Fixierscheibe (5) auf der Trägerplatte platziert werden kann und sich das Multipunktionsventil an einem von der Trägerplatte entfernten Punkt befindet.

4. Magenballonanordnung gemäß Anspruch 1 bis 3, wobei der Ballon aus Silikon biomedizinischer Qualität besteht.

5. Magenballonanordnung gemäß Anspruch 1 bis 4, die weiterhin in der Zone, wo der Ballon und der Katheter mit der Bauchwand in Kontakt sind, einen Film aus einem Material umfasst, das die Fibrose und die Heilung des Gewebes fördern kann.

6. Magenballonanordnung gemäß Anspruch 1 bis 5, die weiterhin eine strahlenundurchlässige Beschichtung des Magenballons für die radiologische Bewertung des in einem Patienten platzierten Ballons umfasst.

7. Magenballonanordnung gemäß Anspruch 1 bis 6, die weiterhin strahlenundurchlässige Marker auf dem Magenballon für die radiologische Bewertung des in einem Patienten platzierten Ballons umfasst.

8. Magenballonanordnung gemäß Anspruch 7, wobei die strahlenundurchlässigen Marker in das Silikon eingearbeitete Metallfilamente sind.

## Revendications

1. Ensemble de ballon intragastrique comportant un ballon intragastrique (1), un élément de traction adapté pour mener le ballon jusqu'à la cavité gastrique du patient à travers la bouche et le tube gastrique de celui-ci, un cathéter de gonflage (2) formé dans son ensemble avec le ballon et, adapté pour être passé à travers la paroi stomacale et les tissus abdominaux, et pour gonfler ledit ballon intragastrique (1) avec un fluide, des éléments de fixation pour fixer ledit ballon (1) dans une position prédéterminée à l'intérieur de la cavité gastrique du patient, de manière à éviter la migration du ballon, et un élément pour commander le volume de gonflage (6) du ballon intragastrique, dans lequel l'ensemble de ballon a, en tant qu'élément de traction simple, un guide de traction (3) formé comme un allongement de l'extrémité distale libre du cathéter de gonflage et adapté pour transporter le ballon vers la cavité gastrique et pour mener ledit cathéter de gonflage à travers la paroi stomacale et d'autres tissus abdominaux, dans lequel les éléments de fixation sont constitués d'une plaque de support (4) avec un seul orifice adapté pour conduire l'extrémité distale du cathéter de gonflage, qui est adaptée pour être fixée sur l'aponévrose antérieure des muscles droits de l'abdomen, et dans lequel l'élément destiné à la commande du volume de gonflage, est constitué d'une valve multiponction gonflante (6), adaptée pour être reliée à l'extrémité distale du cathéter de gonflage (2), en un point supplémentaire de la plaque de support (4), et est adaptée pour être fixée sous la peau du patient sur la région abdominale.

2. Ensemble de ballon intragastrique tel que revendiqué dans la revendication 1, dans lequel la valve multiponction gonflante (6) est adaptée pour être reliée au cathéter de gonflage (2) dans un endroit proche de l'endroit où la plaque de support est disposée, et pour aider, en tant qu'élément de fixation, à fixer le cathéter de gonflage à ladite plaque de support.

3. Ensemble de ballon intragastrique tel que revendiqué dans la revendication 1, comportant en outre une rondelle de fixation (5) ayant un orifice avec un flasque, à travers lequel le cathéter de gonflage (2) peut passer, la rondelle de fixation (5) étant adaptée pour être placée sur la plaque de support, et la valve multiponction étant disposée en un point distant de la plaque de support.

4. Ensemble de ballon intragastrique tel que revendiqué dans les revendications 1 à 3, dans lequel le ballon est constitué de silicone de qualité biomédicale.

5. Ensemble de ballon intragastrique tel que revendiqué dans les revendications 1 à 4, comportant en outre un film en un matériau capable de favoriser la fibrose et la cicatrisation du tissu, dans la zone où le ballon et le cathéter sont en contact avec la paroi abdominale.

6. Ensemble de ballon intragastrique selon les revendications 1 à 5, comportant également un revêtement radio-opaque de ballon intragastrique pour une évaluation radiologique du ballon placé dans un patient.

7. Ensemble de ballon intragastrique selon les revendications 1 à 6, comportant de plus des marqueurs radio-opaques de ballon intragastrique pour une évaluation radiologique du ballon placé dans un patient.

8. Ensemble de ballon intragastrique tel que revendiqué dans la revendication 7, dans lequel les marqueurs radio-opaques sont des filaments métalliques inclus dans le silicone.
